# EUROPEAN PATENT SPECIFICATION

(11) **EP 0 673 243 B1**
(45) Date of publication and mention of the grant of the patent: **06.08.1997**
(21) Application number: 94900974.0
(22) Date of filing: 07.12.1993
(51) Int. Cl.: A61K 9/50, A61K 9/127

(54) **IMPROVING EFFECTIVENESS OF DRUGS**
STEIGERUNG DER WIRKSAMKEIT VON ARZNEISTOFFEN
PROCEDE D'AUGMENTATION DE L'EFFICACITE DE PRINCIPES ACTIFS

(30) Priority: 08.12.1992 GB 9225588
(43) Date of publication of application: 27.09.1995
(73) Proprietor: KAPPA PHARMACEUTICALS LIMITED, Goring-on-Thames Reading RG8 0EN (GB)
(72) Inventor: TAYLOR, Margaret Joan, Leicestershire LE12 5SF (GB)
(74) Representative: McNeight, David Leslie
(86) International application number: GB9302503
(87) International publication number: WO9413270

(56) References cited:
- EP-A- 0 001 104
- WO-A-90/04972

## Description

This invention relates to improving the effectiveness of drugs.

Certain drugs are known to be effective against cancerous tissue, but have severe side effects on healthy tissue. The concept of targeting has been discussed for some time, in which a drug is delivered, usually in minute but effective doses, directly to disease tissue but without also dosing surrounding healthy cells.

Some approaches depend upon the synthesis of new chemicals which, for example, mainly affect cells with a high nucleic acid turnover, due to their fast reproduction. This "magic bullet" or pharmacological approach, however, is difficult where a tumour cell is only quantitatively different from normal cells and has a tendency to produce toxicity.

Another approach is pharmaceutical targeting, in which specially designed delivery devices direct the activity of a non-selective drug. In a "magic gun" system, guidance is imposed on the device such that it permeates only relevant areas, instead of inertly diffusing throughout the tissues until a recognition event occurs.

EP 0 001 104 discloses the irreversible covalent binding of an allosteric effector (inositol hexaphosphate) with the Haemoglobin of isolated anucleate erythrocytes in order to affect the oxygen binding affinity of the Haemoglobin, the allosteric effector being delivered to the erythrocytes by means of lipid vesicles.

A targetted drug can reach widely disseminated cells. Most primary tumours produce secondary or metastatic growths which often prove fatally resistant. A device that killed these growths would be a significant step forward.

An obvious possibility is to harness the cognitive powers of the natural immune system. Tumour cells are known to exhibit surface antigens, which are capable of provoking immunity despite the host's failure to counter a thriving tumour. In this approach, drugs have been attached, by various means, to a specific monoclonal antibody. When in contact with tumour cells in vitro, this worked well, but it failed in vivo.

The present invention provides an effective drug delivery technique which does not have the problems of prior suggestions.

The invention comprises a method for improving the effectiveness of a drug comprising the steps of encapsulating the drug in liposome membranes and loading the thus encapsulated drug into cells extracted from the intended patient, the cells being macrophages or related monocytes, which may be taken from the peritoneum of the patient or from blood.

The extracted cell fraction may be added to a culture medium combined with an agent preventing cell adhesion. Without such an agent, the cells may adhere to the walls of a culture flask, for example, which can make harvesting difficult. Such an agent may comprise PVP coated colloidal silica, which is sold under the name Percoll (RTM) by Pharmacia Inc.

A suitable culture medium is RPMI 1640, and a suitable composition is one part ten-fold strength RPMI 1640 culture medium and nine parts Percoll (RTM).

A cell activator may be added, such as adjuvent peptide or MDP (muramyl dipeptide), with or without the addition of liposomes at this stage. This, especially with liposomes, rapidly activates macrophages to a tumouricidal state.

In any event, the cells are cultured in the composition until activated, as may be determined microscopically by inspection from time to time. This may take several hours at body temperature.

The liposome encapsulated drug may then be added and the composition further incubated until phagocytosis occurs, i.e. the cells imbibe the liposome encapsulated drug. After this, the drug loaded cells may be separated centrifugally and re-suspended into a drug-free saline solution for re-introduction into the patient.

The invention also comprises a kit for carrying out the method above described comprising a culture flask containing a composition of culture medium and cell adhesion preventive agent.

The invention also comprises a composition for carrying out the method above described or for use in a kit as described containing a culture medium and a cell adhesion preventive agent.

Such composition can include also the optional elements referred to above.

Obviously, made-up preparations and culture flasks must all be aseptic, and likewise techniques for introducing the cell fraction and additives.

## Claims

1. A method for improving the effectiveness of a drug comprising the steps of encapsulating the drug in liposome membranes and loading the thus encapsulated drug into cells extracted from the intended patient, the cells being either macrophages or related monocytes.

2. A method according to claim 1, wherein the cells are taken from the peritoneum of the patient or from blood.

3. A method according to any one of the preceding claims, wherein the extracted cell fraction is added to a composition comprising a culture medium combined with an agent preventing cell adhesion.

4. A method according to claim 3, wherein the agent comprises PVP coated colloidal silica.

5. A method according to claim 3, wherein the culture medium is RPMI 1640.

6. A method according to any one of claims 3 to 5, wherein the composition comprises one part ten-fold strength RPMI 1640 culture medium and nine parts PVP coated coloidal silica.

7. A method according to any one of claims 3 to 6, wherein the composition further comprises a cell activator such as adjuvent peptide or muramyl dipeptide (MDP), with or without the addition of liposomes at this stage.

8. A method according to any one of claims 3 to 7, wherein the cells are cultured in the composition until activated.

9. A method according to claim 8, wherein cell activation is determined by microscopial inspection.

10. A method according to any one of claims 3 to 9, wherein the liposome encapsulated drug is incubated in the composition until phagocytosis occurs, i.e. the cells imbibe the liposome encapsulated drug.

11. A method according to claim 10, wherein the drug loaded cells are separated centrifugally and re-suspended into a drug-free saline solution for re-introduction into the patient.

12. A kit for carrying out the method disclosed in claims 1 to 11, comprising a culture flask containing a composition of culture medium and cell adhesion preventive agent.

13. A composition for carrying out the method disclosed in claims 1 to 11 or for use in a kit as described in claim 12 containing a culture medium and a cell adhesion preventive agent.

14. A composition according to claim 13 also comprising a cell activator such as adjuvent peptide or muramyl dipeptide (MDP).

## Patentansprüche

1. Verfahren zum Verbessern der Wirksamkeit eines Arzneimittels, welches die Schritte der Einkapselung des Arzneimittels in Liposom-Membranen und des Einbringens des so eingekapselten Arzneimittels in von dem beabsichtigten Patienten extrahierte Zellen aufweist, wobei die Zellen entweder Makrophagen oder verwandte Monozyten sind.

2. Verfahren nach Anspruch 1, worin die Zellen aus dem Bauchfell des Patienten oder aus Blut genommen sind.

3. Verfahren nach einem der vorhergehenden Ansprüche, worin die extrahierte Zellenfraktion zu einer Zusammensetzung hinzugefügt wird, die ein Kulturmedium in Kombination mit einem eine Zellenadhäsion verhindernden Mittel aufweist.

4. Verfahren nach Anspruch 3, worin das Mittel PVP-beschichtetes kolloidales Siliziumdioxid aufweist.

5. Verfahren nach Anspruch 3, worin das Kulturmedium RPMI 1640 ist.

6. Verfahren nach einem der Ansprüche 3 bis 5, worin die Zusammensetzung ein Teil Kulturmedium aus RPMI 1640 zehnfacher Stärke und neun Teile PVP-beschichtetes kolloidales Siliziumdioxid aufweist.

7. Verfahren nache einem der Ansprüche 3 bis 6, worin die Zusammensetzung weiterhin einen Zellenaktivator wie unterstützendes Peptid oder Muramyl-Dipeptid (MDP) aufweist, mit oder ohne die Hinzufügung von Liposomen in dieser Stufe.

8. Verfahren nach einem der Ansprüche 3 bis 7, worin die Zellen bis zur Aktivierung in der Zusammensetzung kultiviert werden.

9. Verfahren nach Anspruch 8, worin die Zellenaktivierung durch mikroskopische Untersuchung bestimmt wird.

10. Verfahren nach einem der Ansprüche 3 bis 9, worin das liposomengekapselte Arzneimittel in der Zusammensetzung inkubiert wird, bis eine Phagozytose auftritt, d. h. die zellen das liposomengekapselte Arzneimittel aufsaugen.

11. Verfahren nach Anspruch 10, worin die mit dem Arzneimittel befrachteten Zellen zentrifugal getrennt und wieder in einer arzneimittelfreien Kochsalzlösung suspendiert werden für eine Wiedereinführung in den Patienten.

12. Ausrüstung zum Durchführen des in den Ansprüchen 1 bis 11 offenbarten Verfahrens, welche einen Kulturkolben aufweist enthaltend eine Zusammensetzung von Kulturmedium und einem eine Zellenadhäsion verhinderndem Mittel.

13. Zusammensetzung zum Durchführen des in den Ansprüchen 1 bis 11 offenbarten Verfahrens oder zur Verwendung in einer wie in Anspruch 12 beschriebenen Ausrüstung enthaltend ein Kulturmedium und ein eine Zellenadhäsion verhinderndes Mittel.

14. Zusammensetzung nach Anspruch 13, die auch einen zellenaktivator wie unterstützendes Peptid oder Muramyl-Dipeptid (MDP) aufweist.

## Revendications

1. Une méthode pour améliorer l'efficacité d'un médicament, comprenant les étapes d'encapsuler le médicament dans des membranes liposomes et de charger le médicament ainsi encapsulé dans des cellules extraites du patient traité, les cellules étant soit des macrophages, soit des monocytes relatifs.

2. Méthode selon la revendication 1, dans laquelle les cellules sont prises du péritoine du patient ou du sang.

3. Méthode selon l'une quelconque des revendications précédentes, dans laquelle la fraction de cellules extraites est ajoutée à une composition comprenant un milieu de culture associé à un agent qui évite l'adhésion cellulaire.

4. Méthode selon la revendication 3, dans laquelle l'agent comprend de la silice colloïdale enrobée de PVP.

5. Méthode selon la revendication 3, dans laquelle le milieu de culture est le RPMI 1640.

6. Méthode selon l'une quelconque des revendications 3 à 5, dans laquelle la composition comprend une partie de milieu de culture RPMI 1640 à concentration décuple, et neuf parties de silice colloïdale enrobée de PVP.

7. Méthode selon l'une quelconque des revendications 3 à 6, dans laquelle la composition comprend en outre un activateur de cellules tel qu'un peptide adjuvant ou le dipeptide muramyle (MDP), avec ou sans l'addition des liposomes à cette étape.

8. Méthode selon l'une quelconque des revendications 3 à 7, dans laquelle les cellules sont cultivées dans la composition jusqu'à ce qu'elles soient activées.

9. Méthode selon la revendication 8, dans laquelle l'activation des cellules est déterminée par inspection microscopique.

10. Méthode selon l'une quelconque des revendications 3 à 9, dans laquelle le médicament encapsulé par les liposomes est incubé dans la composition jusqu'à ce qu'une phagocytose ait lieu, c'est-à-dire que les cellules s'imprègnent du médicament encapsulé par des liposomes.

11. Méthode selon la revendication 10, dans laquelle les cellules chargées en médicament sont séparées par centrifugation et sont suspendues à nouveau dans une solution salée, libre de médicament, pour une re-introduction dans le patient.

12. Un kit pour la mise en oeuvre de la méthode révélée dans les revendications 1 à 11, comprenant une fiole de culture qui contient une composition de milieu de culture et un agent pour éviter l'adhésion cellulaire.

13. Une composition pour la mise en oeuvre de la méthode révélée dans les revendications 1 à 11, ou pour utilisation dans un kit comme décrit dans la revendication 12 qui contient un milieu de culture et un agent pour éviter l'adhésion cellulaire.

14. Composition selon la revendication 13 qui comprend également un activateur de cellules tel qu'un peptide adjuvant ou le dipeptide muramyle (MDP).
